# EUROPÄISCHE PATENTSCHRIFT

(11) **EP 0 777 647 B1**
(45) Veröffentlichungstag und Bekanntmachung des Hinweises auf die Patenterteilung: **02.12.1998**
(21) Anmeldenummer: 95930475.9
(22) Anmeldetag: 16.08.1995
(51) Int. Cl.: C07C 265/14, C08G 18/77, C08G 18/10

(54) **4,4'-METHYLEN-BIS-(3-CHLOR-2,6-DIALKYLPHENYLISOCYANATE) UND DIESE ENTHALTENDE POLYURETHAN-SYSTEME**
4,4'-METHYLEN-BIS-(3-CHLORO-2,6-DIALKYLPHENYLISOCYANATES) AND POLYURETHANE SYSTEMS CONTAINING THE SAME
4,4'-METHYLEN-BIS-(3-CHLORO-2,6-DIALKYLPHENYLISOCYANATES) ET SYSTEMES DE POLYURETHANNE LES CONTENANT

(30) Priorität: 17.08.1994 CH 2535/94
(43) Veröffentlichungstag der Anmeldung: 11.06.1997
(73) Patentinhaber: LONZA AG, CH-3945 Gampel/Wallis (CH)
(72) Erfinder: DAUM, Ulrich, CH-4114 Hofstetten (CH); HARDT, Peter, CH-3930 Visp (CH)
(74) Vertreter: Weinhold, Peter, Dr.
(86) Internationale Anmeldenummer: EP9503260
(87) Internationale Veröffentlichungsnummer: WO9605171

(56) Entgegenhaltungen:
- EP-A- 0 220 641
- GB-A- 852 651

## Beschreibung

Die Erfindung betrifft 4,4'-Methylen-bis-(3-chlor-2,6-dialkylphenylisocyanate) der allgemeinen Formel 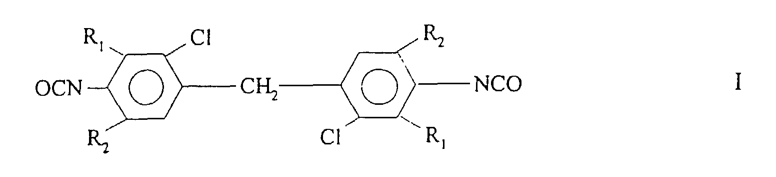 worin R₁ eine Alkylgruppe mit 1 bis 6 C-Atomen und R₂ Chlor oder eine Alkylgruppe mit 1 bis 6 C-Atomen bedeutet, ein Verfahren zur Herstellung der genannten Polyisocyanate sowie die Verwendung der genannten Polyisocyanate in Polyurethan -(PU)-Systemen.

Unter PU-Systemen werden im folgenden Urethan- und / oder Harnstoffgruppen enthaltende Polyurethansysteme verstanden.
Massgebliche Bedeutung als Polyisocyanatkomponenten bei der Herstellung von PU-Systemen haben nach wie vor das Toluol-2,4-diisocyanat und / oder das Toluol-2,6-diisocyanat, kurz als TDI bezeichnet oder das Diphenylmethan-4,4'-diisocyanat, kurz als MDI bezeichnet.

Ein grosser Nachteil von TDI ist seine hohe Toxizität. Obwohl die Verbindung im industriellen Massstab mit grösstmöglicher Sicherheitsvorkehrungen gehandhabt wird, birgt sie ein erhebliches Risikopotential.

Ein vollständiges Ausweichen auf das weniger toxische MDI ist auch nur bedingt möglich, da MDI aufgrund seiner hohen Reaktivität wohl mit Polyolen, nicht aber mit aromatischen Polyaminen verarbeitbar ist.
Die auf TDI und MDI basierenden PU-Systeme sind ausserdem bezüglich ihrer Anwendungstemperatur bei maximal 100 °C limitiert.

Die Aufgabe der vorliegenden Erfindung bestand folglich darin, Polyisocyanate zu entwickeln, die keine hohe Toxizität aufweisen, bezüglich ihrer Reaktivität tiefer als MDI liegen und mit dem klassischen und eingeführten PU-Verarbeitungsverfahren verarbeitbar sind. Mit der Aufgabenstellung verbunden war das Ziel, chemisch beständige PU-Systeme zu entwickeln, die bei Temperaturen über 100 °C angewendet werden können.

Die Aufgabe konnte gelöst werden mit den erfindungsgemässen Polyisocyanaten der genannten allgemeinen Formel I.

R₁ und R₂ in der Bedeutung als C₁-C₆-Alkylgruppe können gleichbedeutend oder verschieden sein und zweckmässig für Methyl, Ethyl, n- oder i-Propyl, n-, i- oder t-Butyl, Pentyl und seine Isomeren und Hexyl und seine Isomeren stehen. Bevorzugt sind R₁ und R₂ gleichbedeutend und stehen für eine der genannten C₁-C₄-Alkylgruppen.

Bevorzugtes Polyisocyanat ist das 4,4'-Methylen-bis-(3-chlor-2,6-diethylphenylisocyanat) mit der Bedeutung von R₁ und R₂ = Ethyl.

Die Herstellung der erfindungsgemässen Polyisocyanate erfolgt auf bekannte Weise durch Umsetzung des entsprechenden Polyamins mit Phosgen oder einer Phosgen-freisetzenden Verbindung wie z. B. Di- oder Triphosgen (vgl. z. B. Ullmanns Encykl. d. techn. Chemie, 4. Aufl., Bd. 13, S. 351ff.).
Die entsprechenden Polyamine sind in der europäischen Anmeldung EP-A 0 220 641 beschrieben. Bevorzugtes Polyamin ist das 4,4'-Methylen-bis-(3-chlor-2,6-diethylanilin) (M-CDEA).
Die Phosgenierung erfolgt zweckmässig in Gegenwart eines inerten Lösungsmittels wie z. B. Toluol oder Chlorbenzol bei erhöhter Temperatur. Die Umsetzung verläuft in der Regel praktisch quantitativ.
Die resultierenden Polyisocyanate zeichnen sich durch eine hohe Reinheit aus.

Die Verarbeitung der erfindungsgemässen Polyisocyanaten zu PU-Systemen erfolgt grundsätzlich auf bekannte Weise durch Umsetzung mit Verbindungen mit mindestens zwei gegenüber Polyisocyanaten aktiven Wasserstoffatomen und gegebenenfalls Kettenverlängerungsmitteln und gegebenenfalls in Gegenwart der üblichen Katalysatoren und gegebenenfalls weiteren Zusätzen (vgl. Saechtling, Kunststoff Taschenbuch, 24. Ausgabe, erschienen in Carl Hauser Verlag, München 1989, S. 429ff.).

Es ist ebenso möglich, Mischungen der erfindungsgemässen Polyisocyanate mit anderen aliphatischen oder aromatischen Polyisocyanaten oder Prepolymere der Polyisocyanate bzw. Prepolymere basierend auf Mischungen der Polyisocyanaten mit aliphatischen oder aromatischen Polyisocyanaten einzusetzen.

Geeignete Vertreter von Verbindungen mit mindestens zwei gegenüber Polyisocyanaten aktiven Wasserstoffatomen sind insbesondere Polyole wie z. B. Polyetherpolyole, Polyesterpolyole oder sonstige Polyole (z. B. Polycaprolactone). Geeignete Vertreter von Kettenverlängerungsmitteln sind Polyamine wie z. B. die aromatischen Diamine MOCA, M-CDEA, Gemische von M-CDEA mit aromatischen oder aliphatischen Diaminen oder Polyolen oder Isomerengemische von Dimethylthiotoluoldiamin (ibid. S. 430 oder EP-A 220 641).

Ausserdem sind alle üblichen Katalysatoren wie Tetramethylbutandiamin (TMBDA), Diazabicyclooctan (DABCO), Dibutylzinndilaurat (DBTC) oder organische Schwermetallverbindungen einzeln oder in Kombination mit Zusätzen wie z. B. Weichmacher, Stabilisatoren, Flammschutzmitteln, Treibmitteln oder Füllstoffen anwendbar (ibid. S. 430).

Ein grosser Vorteil der erfindungsgemässen Polyisocyanate besteht in deren Verarbeitbarkeit in den gängigen PU-Verarbeitungsverfahren wie z. B. dem one-shot RIM-Verfahren, dem two-shot Prepolymerverfahren oder dem two-shot Direktverfahren.
Der bevorzugten Anwendung der Polisocyanate im PU-Elastomer Sektor oder insbesondere im PU-Giesselastomer Sektor entsprechend, wird dem Prepolymerverfahren den Vorzug gegeben.

Die erfindungsgemässen Polyisocyanate werden zweckmässig eingesetzt in ein Polyurethan-System, das herstellbar ist durch Umsetzung von einem
a) 4,4'-Methylen-bis-(3-chlor-2,6-dialkylphenylisocyanat) der allgemeinen Formel I mit
b) Verbindungen mit mindestens zwei gegenüber Isocyanaten aktiven Wasserstoffatomen und gegebenenfalls
c) Kettenverlängerungsmitteln gegebenenfalls in Gegenwart der üblichen Katalysatoren und gegebenenfalls weiteren Zusätzen.

Bevorzugt ist ein Polyurethansystem, das herstellbar ist durch Umsetzung von einem
a) 4,4'-Methylen-bis-(3-chlor-2,6-dialkylphenylisocyanat) der allgemeinen Formel I mit
b) Verbindungen mit mindestens zwei gegenüber Isocyanaten aktiven Wasserstoffatomen, bevorzugt die zuvor beschriebenen und
c) einem aromatischen Diamin als Kettenverlängerer in Gegenwart der genannten üblichen Zusätze.

Besonders bevorzugt wird als aromatisches Diamin ein 4,4'-Methylen-bis-(3-chlor-2,6-dialkylanilin), speziell das 4,4'-Methylen-bis-(3-chlor-2,6-diethylanilin), entweder einzeln oder als Bestandteil eines Gemisches mit weiteren aromatischen oder aliphatischen Diaminen oder mit Polyolen und als Komponente a) das 4,4'-Methylen-bis-(3-chlor-2,6-diethylphenylisocyanat) angewendet.

Die auf Basis der neuen und erfindungsgemässen Polyisocyanate hergestellten PU-Systeme zeichnen sich aus durch eine, im Vergleich zu bekannten PU-Systemen nicht erwartete hohe chemische Beständigkeit und durch eine Anwendungstemperatur bis 180 °C.

Diese erfindungsgemässen PU-Systeme werden daher vor allem im PU-Elastomer - insbesondere im Giesselastomer-Sektor zur Herstellung von z. B. Rollen, Rädern, Walzenbezügen, Isolatoren, Dichtungen oder Vergussmassen verwendet. Es ist aber durchaus möglich die PU-Systeme für das Spraycoating oder für PU-Schäume einzusetzen.

### Beispiele

### Beispiel 1a

### Herstellung von 4,4'-Methylen-bis-(3-chlor-2,6-diethylphenylisocyanat)

100 g (0,26 mol) 4,4'-Methylen-bis-(3-chlor-2,6-diethylanilin) wurden bei Raumtemperatur in 1000 g Dichlorbenzol im Autoklaven vorgelegt. In dieser Lösung wurden 57 g (0,58 mol) Phosgen währen 30 Minuten eingeleitet. Das Reaktionsgemisch wurde darauf im geschlossenen Autoklaven bei 80 °C während 1 Stunde gerührt. Dann wurde entspannt und der entstandene Chlorwasserstoff, das überschüssige Phosgen und das Lösungsmittel entfernt. Dabei resultierte das Titelprodukt in einer Ausbeute von 110 g (98% der Theorie).
- IR (KBr):: 2288.1 cm⁻¹
- ¹H-NMR (CDCl₃, 400 MHz) in ppm:: 6,69 s, 2H;
4,12 s, 2H;
2,91 q, 4H, J= 7,5 Hz;
2,59 q, 4H, J= 7,6 Hz;
1,20 t, 6H, J= 7,6 Hz;
1,15 t, 6H, J= 7,5 Hz.

### Beispiel 1b

In Analogie zu Beispiel 1a, aber mit dem Lösungsmittel Toluol, wurde das Titelprodukt in einer Ausbeute von 109 g erhalten.

### Prüfung von 4,4'-Methylen-bis-(3-chlor-2,6-diethylphenylisocyanat) im Vergleich mit Isocyanaten aus dem Stand der Technik in PU-Systemen.

### 1. Eingesetzte Isocyanate

- MCDE-I: 4,4'-Methylen-bis-(3-chlor-2,6-diethylphenylisocyanat) = erfindungsgemässe Verbindung
- MDE-I: 4,4'-Methylen-bis-(2,6-diethylphenylisocyanat) = Vergleichssubstanz
- MDI: 4,4'-Methylen-bis-phenylisocyanat = Vergleichssubstanz

### 2. Herstellung der Prepolymere (Komponente A)

### Prepolymer 1 (Erfindung)

Prepolymer auf Basis Polytetramethylenetherglykol (PTMG; Tetrathane 650, Du Pont) mit Mol. Gew. 650 und MCDE-I.

1850 g = 4 mol 94%iges MCDE-I wurden unter Stickstoff (N₂) bei 80 °C aufgeschmolzen, im Reaktionskolben vorgelegt und unter Rühren mit 1300 g PTMG = 2 mol innert 30 Minuten versetzt. Das PTMG ist linear und wurde vor der Zugabe zum Isocyanat 1 Stunde bei 100 °C und einem Vakuum von 2500 Pa entwässert. Nach der vollständigen Zugabe des PTMG wurde noch anschliessend 2 Stunden bei 90 °C unter N₂ gerührt.

Man erhielt so ein Prepolymer mit einem Gehalt von 5,2% an freien NCO-Gruppen. Wir bezeichnen dieses Prepolymer als "PTMG 650-MCDE-I".

### Prepolymer 2 (Erfindung)

Prepolymer auf Basis Polytetramethylenetherglykol (PTMG; Terathane 2000, Du Pont) mit Mol. Gew. 2000 und MCDE-I.

1234 g = 2,67 mol 95%iges MCDE-I wurden unter N₂ bei 80 °C aufgeschmolzen, im Reaktionskolben vorgelegt und unter Rühren mit 2133 g = 1,066 mol PTMG innert 30 Minuten versetzt. Das PTMG ist linear und wurde vor der Zugabe zum Isocyanat 1 Stunde bei 100 °C und einem Vakuum von 2500 Pa entwässert. Nach der vollständigen Zugabe des PTMG wurde noch anschliessend 2 Stunden bei 90 °C unter N₂ gerührt.

Man erhielt so ein Prepolymer mit einem Gehalt von 3,96% an freien NCO-Gruppen. Wir bezeichnen dieses Prepolymer als "PTMG 2000-MCDE-I".

### Prepolymer 3 (Erfindung)

Prepolymer auf Basis Polycaprolactonglykol (PCL; CAPA 220, Interox) mit Mol. Gew. 2000 und MCDE-I.

1245 g = 2,7 mol 94%iges MCDE-I wurden unter N₂ bei 80 °C aufgeschmolzen, im Reaktionskolben vorgelegt und unter Rühren mit 2133 g = 1,066 mol PCL innert 30 Minuten versetzt. Das PCL ist linear und wurde vor der Zugabe zum Isocyanat 1 Stunde bei 100 °C und einem Vakuum von 2500 Pa entwässert. Nach der vollständigen Zugabe des PCL wurde noch anschliessend 2 Stunden bei 90 °C unter N₂ gerührt.
Man erhielt so ein Prepolymer mit einem Gehalt von 3,92% an freien NCO-Gruppen. Wir bezeichnen so ein Prepolymer als "PCL 2000-MCDE-I".

### Prepolymer 4 (Vergleich)

Prepolymer auf Basis PTMG (Terathane 2000, Du Pont) mit Mol. Gew. 2000 und MDE-I.

1158 g = 3 mol 94%iges MDE-I wurden unter N₂ bei 80 °C aufgeschmolzen im Reaktionskolben vorgelegt und unter Rühren mit 2400 g = 1,2 mol PTMG innert 30 Minuten versetzt. Das PTMG ist linear und wurde vor der Zugabe zum Isocyanat 1 Stunde bei 100 °C und einem Vakuum von 2500 Pa entwässert. Nach der vollständigen Zugabe des PTMG wurde noch anschliessend 2 Stunden bei 90 °C unter N₂ gerührt.

Man erhielt so ein Prepolymer mit einem Gehalt von 4,32% an freien NCO-Gruppen. Wir bezeichnen dieses Prepolymer als "PTMG 2000-MDE-I".

### Prepolymer 5 (Vergleich)

Prepolymer auf Basis PCL (CAPA 220, Interox) mit Mol. Gew. 2000 und MDE-I.

Ersetzt man in Prepolymer 4 das PTMG durch die gleiche Menge PCL, so erhält man bei sonst identischen Bedingungen ein Prepolymer mit einem Gehalt von 4,23% an freien NCO-Gruppen. Wir bezeichnen dieses Prepolymer als "PCL 2000-MDE-I".

### Prepolymer 6

Prepolymer auf Basis PCL (CAPA 220, Interox) mit Mol. Gew. 2000 und MDI.

400 g = 1,6 mol MDI wurden unter N₂ bei 60 °C aufgeschmolzen, im Reaktionskolben vorgelegt, auf 80 °C erhitzt und unter Rühren mit 1000 g = 0,5 mol PCL innert 10 Minuten versetzt. Das PCL ist linear und wurde vor der Zugabe zum Isocyanat 1 Stunden bei 100 °C und einem Vakuum von 2500 Pa entwässert. Nach der vollständigen Zugabe des PCL wurde noch anschliessend 2 Stunden bei 80 °C unter N₂ gerührt.
Man erhielt ein Prepolymer mit einem Gehalt von 6,6% an freien NCO-Gruppen. Wir bezeichnen dieses Prepolymer als "PCL 2000-MDI".

### 3. Komponente B

Die Komponente B ist entweder das geschmolzene Diamin* oder die klare entgaste, auf Verarbeitungstemperatur (80 °C) abgekühlte Lösung des betreffenden Diamins bzw. Diamingemisches im entsprechenden Polyol. Die Lösungen im Polyol enthalten zudem bezogen auf das Gesamtsystem (Komponente A + B) 0,3% einer organischen Wismuthverbindung (Coscat® 83 Catalyst der CasChem. Inc., New Jersey).
* I 4,4'-Methylen-bis-(3-chlor-2,6-diethylanilin) M-CDEA
II Diamingemisch, Luvocure MUT-HT, Lehmann & Voss
III Gemisch der 2,4 und 2,6 Isomere von Dimethylthiotoluoldiamin, Ethacure 300, Albemarle Inc.

### 4. Bereitung der Prüfkörper

Das Prepolymer (Komponente A) und die Diamine (Kettenverlängerer; Komponente B) wurden im molaren Verhältnis 1:0,95, d. h. NCO-Gruppen zur Summe von freien OH- und NH₂-Gruppen, bei 80 °C während 30 Sekunden innig vermischt, in eine auf 110 °C vorgewärmte Metallform mit den Innenmassen von 200*200*2 (in mm) gegossen und schliesslich beim Beginn des Gelierens (Topfzeit) in einer Presse bei 200 bar und 110 °C gepresst. Nach vollständiger Durchhärtung (Entformzeit) wurde entformt und bei 110 °C 16 Stunden nachgetempert. Aus den gehärteten Elastomeren wurden Prüfkörper herausgestanzt.

### 5. Prüfparameter:

| | |
|---|---|
| Härte Shore A und Shore D | DIN 53505 |
| Weiterreisswiderstand [N/mm] | DIN 53515 |
| Zugfestigkeit [N/mm²] | DIN 53504 |
| Spannung bei 100 % Dehnung | DIN 53504 |
| Bruchdehnung % | DIN 53504 |

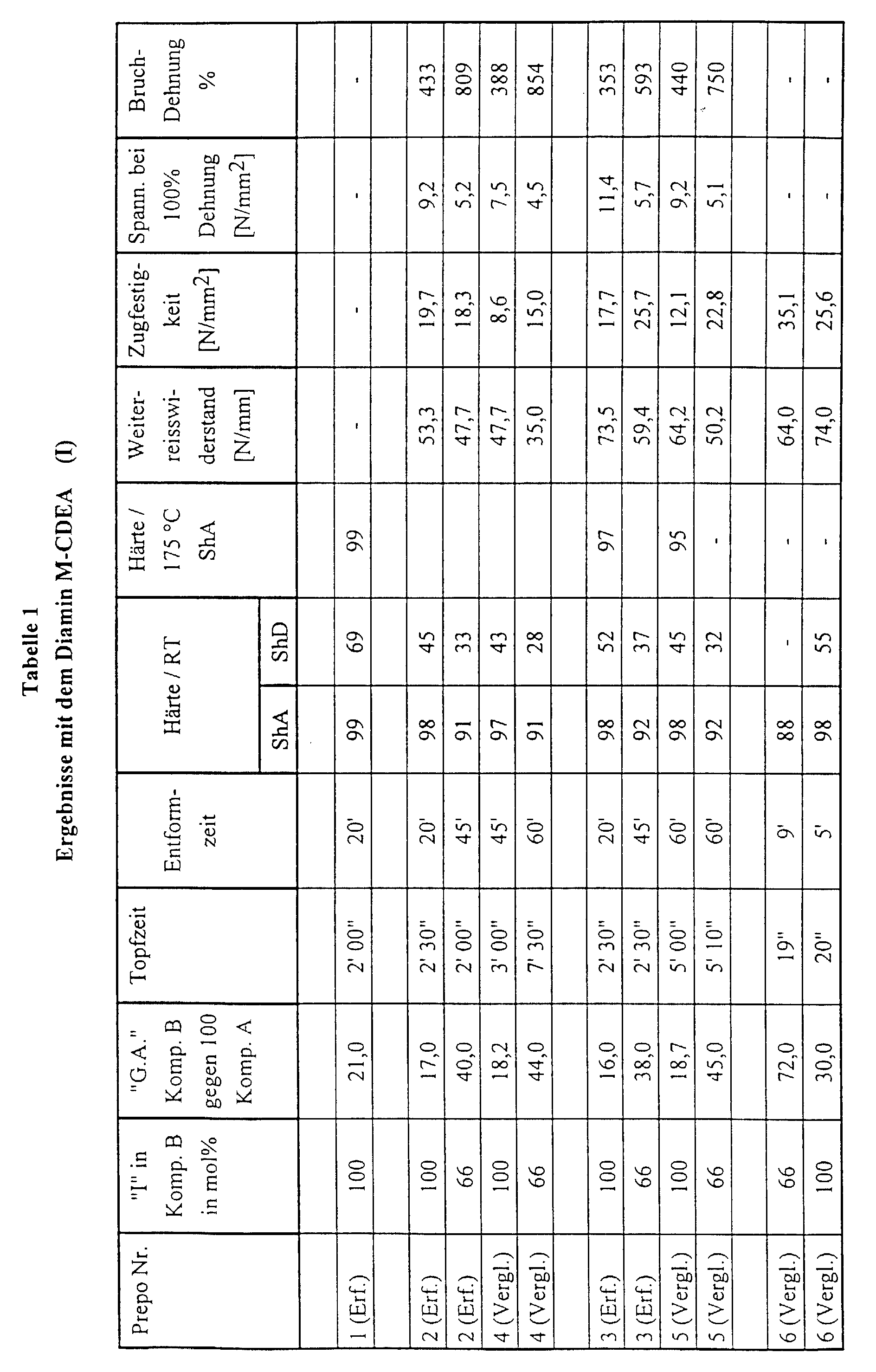

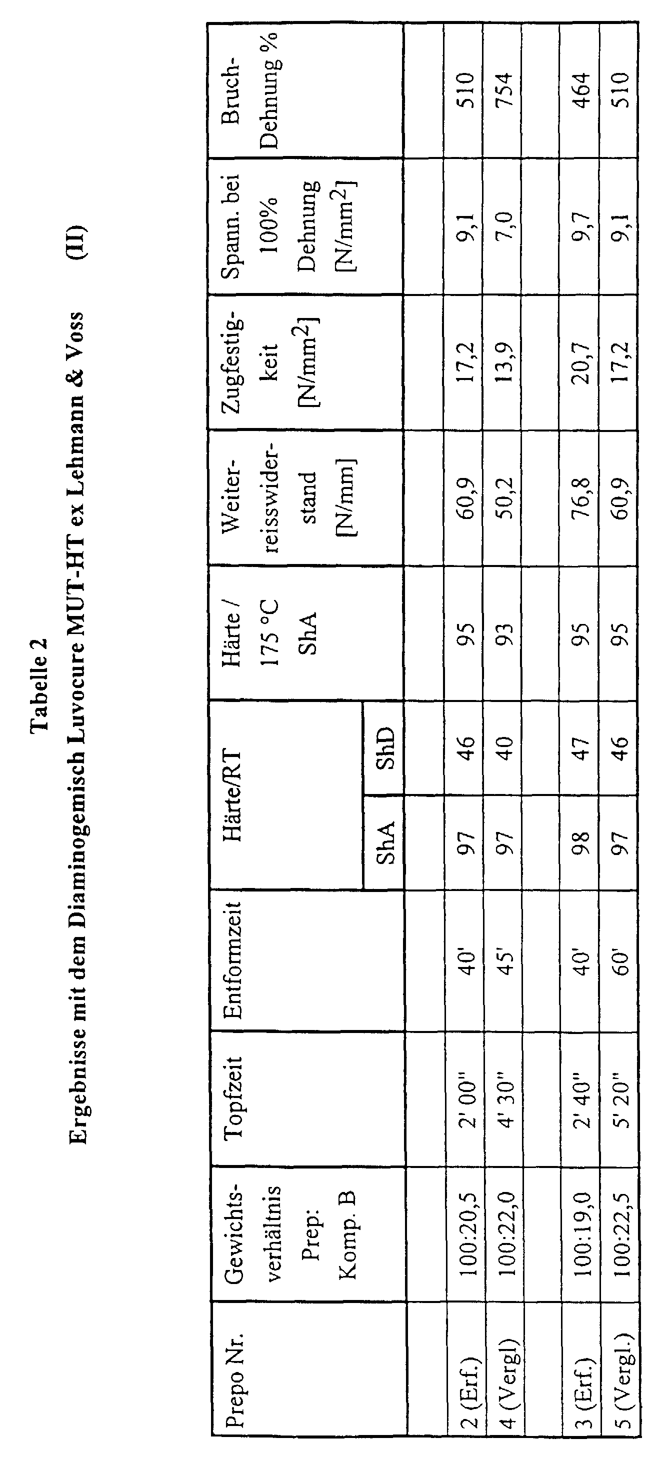

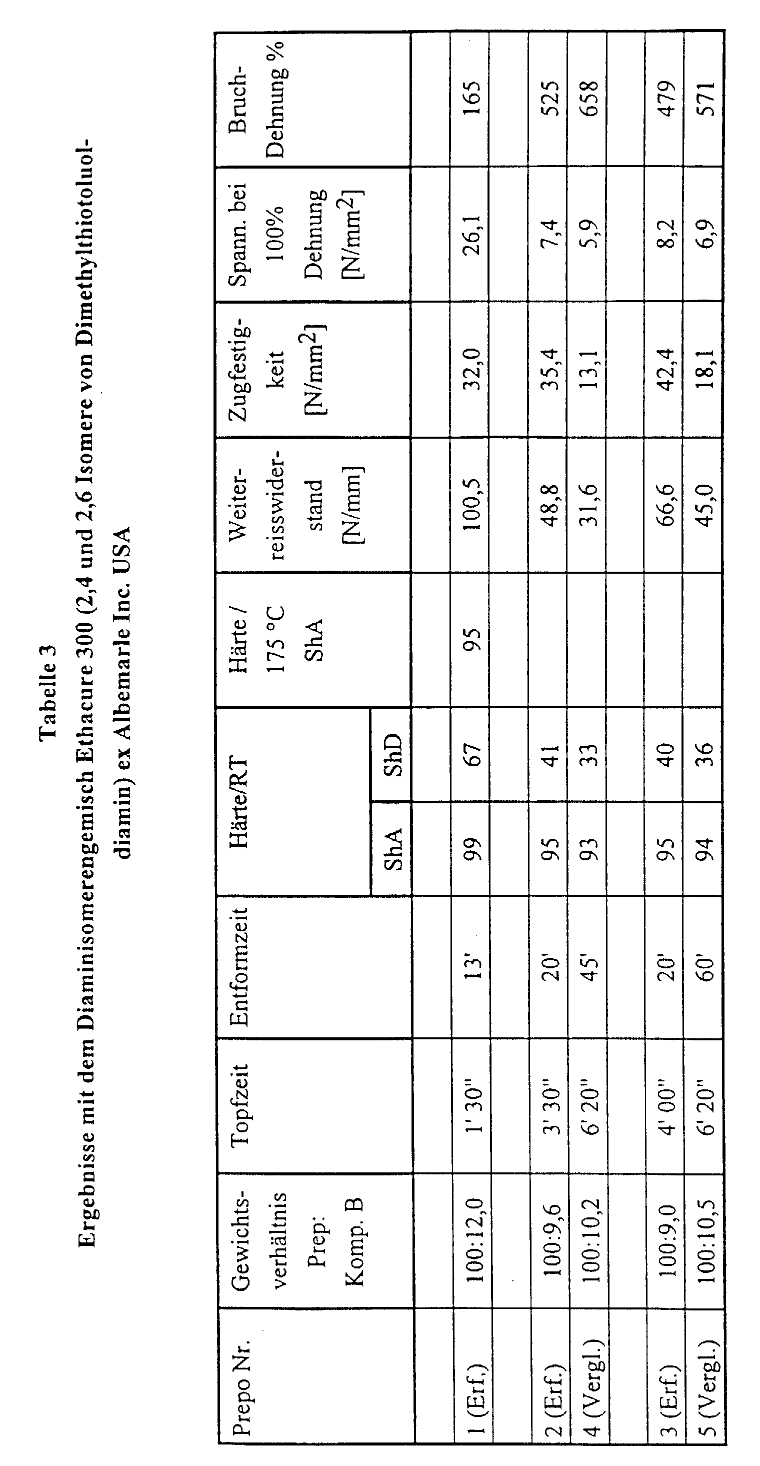

## Patentansprüche

1. 4,4'-Methylen-bis-(3-chlor-2,6-dialkylphenylisocyanate) der allgemeinen Formel 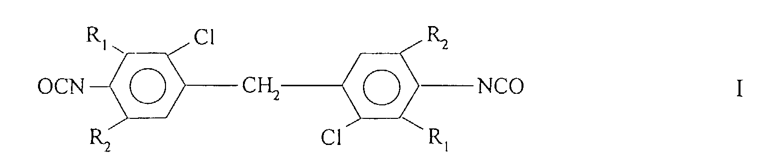 worin R₁ eine Alkylgruppe mit 1 bis 6 C-Atomen und R₂ Chlor oder eine Alkylgruppe mit 1 bis 6 C-Atomen bedeutet.

2. 4,4'-Methylen-bis-(3 chlor-2,6-diethylphenylisocyanat).

3. Verfahren zur Herstellung von 4,4'-Methylen-bis-(3-chlor-2,6-dialkylphenylisocyanaten) der allgemeinen Formel 1, dadurch gekennzeichnet, dass ein 4,4'-Methylen-bis-(3-chlor-2,6-dialkylanilin) der allgemeinen Formel 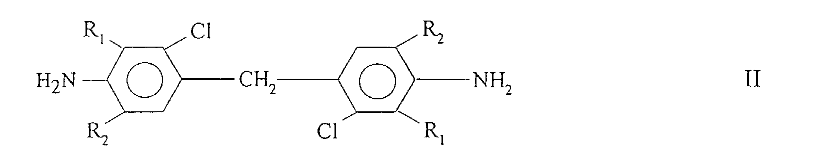 worin R₁ und R₂ die genannte Bedeutung haben, mit Phosgen oder einer Phosgen-freisetzenden Verbindung umgesetzt wird.

4. Verwendung der 4,4'-Methylen-bis-(3-chlor-2,6-dialkylphenylisocyanate) der allgemeinen Formel I zur Herstellung von Polyurethan-Systemen.

5. Verwendung nach Patentanspruch 4, dadurch gekennzeichnet, dass 4,4'-Methylen-bis-(3-chlor-2,6-diethylphenylisocyanat) verwendet wird.

6. Polyurethan-System,herstellbar durch Umsetzung von
a) einem 4,4'-Methylen-bis-(3-chlor-2,6-dialkylphenylisocyanat) der allgemeinen Formel I mit
b) Verbindungen mit mindestens zwei gegenüber Isocyanaten aktiven Wasserstoffatomen und gegebenenfalls
c) Kettenverlängerungsmitteln, gegebenenfalls in Gegenwart der üblichen Katalysatoren und gegebenenfalls weiteren Zusätzen.

7. Polyurethan-System nach Patentanspruch 6, herstellbar durch Umsetzung von
a) einem 4,4'-Methylen-bis-(3-chlor-2,6-dialkylphenylisocyanat) mit
b) Verbindungen mit mindestens zwei gegenüber Isocyanaten aktiven Wasserstoffatomen und
c) einem aromatischen Diamin als Kettenverlängerungsmittel, gegebenenfalls in Gegenwart der üblichen Katalysatoren und gegebenenfalls weiteren Zusätzen.

8. Polyurethan-System nach Patentanspruch 7, dadurch gekennzeichnet, dass als aromatisches Diamin ein 4,4'-Methylen-bis-(3-chlor-2,6-dialkylanilin) oder ein Gemisch von einem 4,4'-Methylen-bis-(3-chlor-2,6-dialkylanilin) mit einem oder mehreren aromatischen oder aliphatischen Diaminen oder einem Polyol eingesetzt wird.

9. Polyurethan-System nach Patentanspruch 7 oder 8, dadurch gekennzeichnet, dass 4,4'-Methylen-bis-(3-chlor-2,6-diethylphenylisocyanat) und als aromatisches Diamin 4,4'-Methylen-bis-(3-chlor-2,6-diethylanilin) oder ein Gemisch von 4,4'-Methylen-bis-(3-chlor-2,6-diethylanilin) mit einem oder mehreren aromatischen oder aliphatischen Diaminen oder einem Polyol eingesetzt wird.

## Claims

1. 4,4'-Methylene-bis-(3-chloro-2,6-dialkylphenyl isocyanates) of the general formula 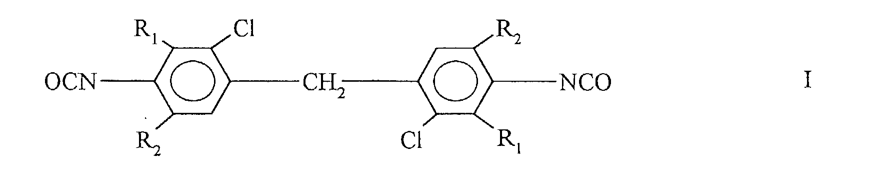 where R₁ is an alkyl group having from 1 to 6 carbon atoms and R₂ is chlorine or an alkyl group having from 1 to 6 carbon atoms.

2. 4,4'-Methylene-bis-(3 -chloro-2,6-diethylphenyl isocyanate).

3. Process for preparing 4,4'-methylene-bis-(3-chloro-2,6-dialkylphenyl isocyanates) of the general formula I, characterized in that a 4,4'-methylene-bis-(3-chloro-2,6-dialkylaniline) of the general formula 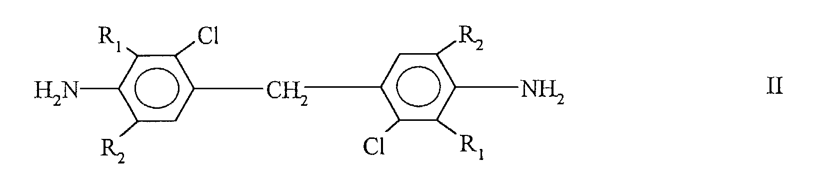 where R₁ and R₂ have the said meanings, is reacted with phosgene or a phosgeneliberating compound.

4. Use of the 4,4'-methylene-bis-(3-chloro-2,6-dialkylphenyl isocyanates) of the general formula I for preparing polyurethane systems.

5. Use according to Claim 4, characterized in that 4,4'-methylene-bis-(3-chloro-2,6-diethylphenyl isocyanate) is used.

6. Polyurethane system which can be prepared by reacting
a) a 4,4'-methylene-bis-(3-chloro-2,6-dialkylphenyl isocyanate) of the general formula I with
b) compounds containing at least two hydrogen atoms reactive towards isocyanates, and, if desired,
c) chain extenders, if desired in the presence of the customary catalysts and, if desired, further additives.

7. Polyurethane system according to Claim 6 which can be prepared by reacting
a) a 4,4'-methylene-bis-(3-chloro-2,6-dialkylphenyl isocyanate) with
b) compounds containing at least two hydrogen atoms reactive towards isocyanates and
c) an aromatic diamine as chain extender, if desired in the presence of the customary catalysts and, if desired, further additives.

8. Polyurethane system according to Claim 7, characterized in that the aromatic diamine used is a 4,4'-methylene-bis-(3-chloro-2,6-dialkylaniline) or a mixture of a 4,4'-methylene-bis-(3-chloro-2,6-dialkylaniline) with one or more aromatic or aliphatic diamines or a polyol.

9. Polyurethane system according to Claim 7 or 8, characterized in that 4,4'-methylene-bis-(3-chloro-2,6-diethylphenyl isocyanate) is used and the aromatic diamine used is 4,4'-methylene-bis-(3-chloro-2,6-diethylaniline) or a mixture of 4,4'-methylene-bis-(3-chloro-2,6-diethylaniline) with one or more aromatic or aliphatic diamines or a polyol.

## Revendications

1. 4,4'-méthylèn-bis-(3-chlor-2,6-dialkylphénylisocyanates) de la formule générale 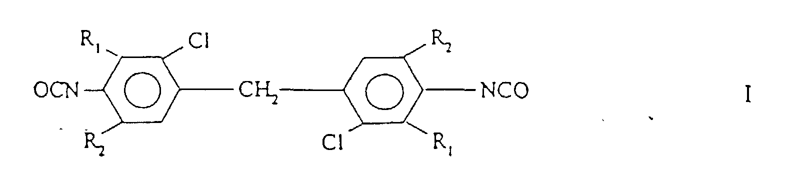 dans laquelle R₁ signifie un groupe alkyle avec 1 à 6 atomes C et R₂ le chlore ou un groupe alkyle avec 1 jusqu'à 6 atomes C.

2. 4,4'-méthylèn-bis-(3-chlor-2,6-diéthylphénylisocyanate).

3. Procédé pour la préparation de 4,4'-méthylèn-bis-(3-chlor-2,6-dialkylphénylisocyanates) de la formule générale I, caractérisé en ce que l'on met en réaction une 4,4'-méthylèn-bis-(3-chlor-2,6-dialkylaniline) de la formule générale 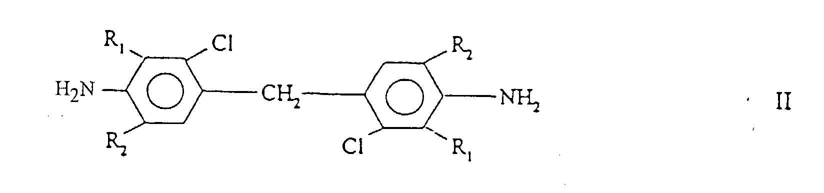 dans laquelle R₁ et R₂ ont la signification ci-dessus, avec du phosgène ou un composé libérant du phosgène.

4. Utilisation du 4,4'-méthylèn-bis-(3-chlor-2,6-dialkylphénylisocyanates) de la formule générale I pour la préparation de systèmes de polyuréthanne.

5. Utilisation selon la revendication 4, caractérisée en ce que l'on utilise le 4,4'-méthylèn-bis-(3-chlor-2,6-diéthylphénylisocyanate).

6. Système de polyuréthanne pouvant être préparé par mise en réaction de
a) un 4,4'-méthylèn-bis-(3-chlor-2,6-dialkylphénylisocyanate) de la formule générale I avec
b) des composés ayant au moins deux atomes d'hydrogène actifs vis-à-vis des isocyanates et éventuellement
c) des agents de prolongation de chaîne, éventuellement en présence des catalyseurs usuels et éventuellement d'autres additifs.

7. Système de polyuréthanne selon la revendication 6, pouvant être préparé par mise en réaction de
a) un 4,4'-méthylèn-bis-(3-chlor-2,6-dialkylphénylisocyanate) avec
b) des composés ayant au moins deux atomes d'hydrogène actifs vis-à-vis des isocyanates et
c) une diamine aromatique en tant qu'agent de prolongation de chaîne, éventuellement en présence des catalyseurs usuels et éventuellement d'autres additifs.

8. Système de polyuréthanne selon la revendication 7, caractérisé en ce qu'en tant que diamine aromatique, on utilise une 4,4'-méthylèn-bis-(3-chlor-2,6-dialkylaniline) ou un mélange de 4,4'-méthylèn-bis-(3-chlor-2,6-dialkylaniline) avec une ou plusieurs diamines aromatiques ou aliphatiques ou un polyol.

9. Système de polyuréthanne selon la revendication 7 ou 8, caractérisé en ce que l'on utilise le 4,4'-méthylèn-bis-(3-chlor-2,6-diéthylphénylisocyanate) et en tant que diamine aromatique, la 4,4'-méthylèn-bis-(3-chlor-2,6-diéthylaniline) ou un mélange de 4,4'-méthylèn-bis-(3-chlor-2,6-diéthylaniline) avec une ou plusieurs diamines aromatiques ou aliphatiques ou un polyol.
